(19) Europäisches Patentamt
European Patent Office
Office européen des brevets

(11) Publication number: **0 075 452 B1**

# (12) EUROPEAN PATENT SPECIFICATION

(45) Date of publication of patent specification: **02.07.86**

(51) Int. Cl.[4]: **C 07 D 501/34,** A 61 K 31/545

(21) Application number: **82304896.2**

(22) Date of filing: **16.09.82**

## (54) Cephalosporin derivatives.

(30) Priority: **18.09.81 JP 147578/81**
**22.09.81 JP 149870/81**

(43) Date of publication of application:
**30.03.83 Bulletin 83/13**

(45) Publication of the grant of the patent:
**02.07.86 Bulletin 86/27**

(84) Designated Contracting States:
**BE CH DE FR GB IT LI SE**

(56) References cited:
**US-A-3 641 015**

(73) Proprietor: **KUREHA KAGAKU KOGYO KABUSHIKI KAISHA**
**9-11 Horidome-cho 1-chome Nihonbashi**
**Chuo-ku**
**Tokyo (JP)**

(72) Inventor: **Muto, Shigeaki**
**3-23-2, Higashi-Horikiri**
**Katsushika-ku Tokyo (JP)**
Inventor: **Niimura, Kouichi**
**31-201 Shinsayama-Haitsu 63 Aoyagi**
**Sayama-shi Saitama-ken (JP)**
Inventor: **Ando, Takao**
**No. 24 Daiichi-Kurehasoh**
**3-10-13 Nerima Nerima-ku Tokyo (JP)**
Inventor: **Fujii, Masahiko**
**202 Mezon Shirayuri 1-6-11 Nakaizumi**
**Komae-shi Tokyo (JP)**
Inventor: **Furusho, Takao**
**1-6-13 Asahi-machi**
**Machida-shi Tokyo (JP)**
Inventor: **Yoshikumi, Chikao**
**2-19-46 Higashi**
**Kunitachi-shi Tokyo (JP)**
Inventor: **Kanno, Akihiko**
**5-31-1 Matsubara**
**Setagaya-ku Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(74) Representative: **Myerscough, Philip Boyd et al**
**J.A.Kemp & Co. 14, South Square Gray's Inn**
**London, WC1R 5EU (GB)**

**Description**

The present invention relates to cephalosporin compounds, their preparation and pharmaceutical compositions containing them.

Antibiotics which are so-called cephalosporins (hereinafter referred to as a cephalosporin antibiotic) are well known as excellent drugs due to their selective toxicity to bacteria. However, such cephalosporin antibiotics have a serious defect, that is the may disturb the beneficial bacterial colonies ordinarily present in living bodies, particularly the intestinal bacterial colonies, since they may also be antibacterially active against the beneficial bacteria. This defect is very serious when such a cephalosporin antibiotic is orally administered. As a result, "microbisme selectionné et substitué" is caused resulting in colitis and diarrhoea.

The present invention is concerned with antibiotics which do not have such a defect. In particular, the invention relates to a compound obtained by modifying chemically a cephalosporin, antibacterial activity being reduced by such chemical modification but recovered when the compound is absorbed from the intestinal tract. The compounds of the invention can be formulated into pharmaceutical compositions and exhibit an activity similar to a cephalosporin in a living body.

The cephalosporin derivatives of the invention have the ganeral formula (I):

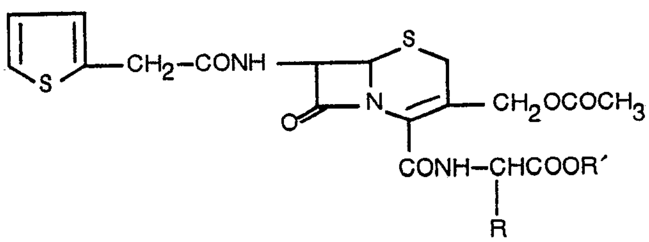

(I)

wherein R′ is a hydrogen atom, an alkyl group having 1 to 4 carbon atoms or a pharmaceutically acceptable cation, preferably an alkali metal cation, and R is

$-CH_2COOR'$, $-CH_2CH_2SR'$, 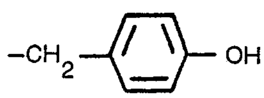, $-CH_2CH_2COOR'$, or $-CH(CH_3)_2$

wherein R′ is as defined above. The carboxyl and the thiol groups of the cephalosporin derivative may be in the form of a salt or a thioalcoholate other than an alkali metal salt, for example an alkali earth metal salt, an aluminium salt or an ammonium salt. The compounds of formula (I), and indeed all cephalosporin derivatives described and illustrated by formulae herein, have the usual absolute cephalosporin stereochemistry of 6R:7R.

The cephalosporin derivatives of the invention may be derived from a cephalosporin antibiotic by a chemical modification. The cephalosporin derivatives are absorbed from the intestinal tract without affecting the bacterial colonies ordinarily present in living bodies and show an antibacterial activity when entering into blood in the living bodies. Therefore, the cephalosporin derivatives of the invention are an antibiotic quite different from the conventional cephalosporin antibiotics.

The cephalosporin derivative of the invention may be prepared by the following process.

7-(thienyl-2-acetamido)cephalosporanic acid having the formula (II);

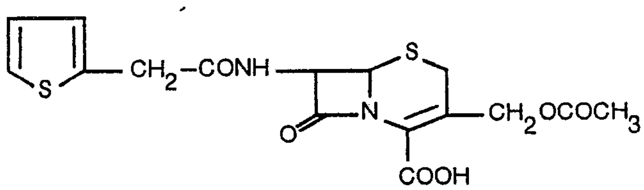

(II)

or a salt or a chloride thereof is dissolved in an organic solvent, for example, dimethylformamide(DMF), acetone, benzene, dichloromethane, pyridine, tetrahydrofuran (THF), dioxane, triethylamine, and the like. It is preferable to add an activating agent to the system, for example, carbondiimide, ethyl chloroformate, oxalyl chloride, and the like. Into the obtained solution, an amine compound having the general formula (III);

$$NH_2—\underset{\displaystyle R}{\underset{|}{C}}HCOOR' \quad (III)$$

wherein R′ and R are defined above is added and the reaction is carried out at the temperature of −30°C to

50°C for 0.5 to 48 hours. The cephalosporin derivative of the invention is thereafter collected by the conventional method, for example, washing with a solvent, extraction with a solvent, recrystallization, and the like.

As seen from the Examples described hereinafter, the cephalosporin derivative of the invention has low toxicity and exhibits an antibacterial activity when absorbed from the intestinal tract without affecting the intestinal bacterial colonies.

The cephalosporin derivative of the invention may be useful in the same field as the conventional cephalosporin antibiotics since the cephalosporin derivative is transformed into a cephalosporin antibiotic when absorbed from the intestinal tract.

The cephalosporin derivative of the invention may be used singly as an antibacterial drug.

The cephalosporin derivative of the invention may be used as an active ingredient of a pharmaceutical composition. The pharmaceutical composition contains a pharmaceutically acceptable carrier, diluent or adjuvent as well as at least one cephalosporin derivative of the invention and may be used in a dosage unit form. The composition may be administered orally or rectally or by injection. The dosage form for oral administration may be tablet, capsule, powder, granule, pill, ampoule, or the like.

The pharmaceutical composition may contain filler, extender, binder, wetting agent, disintegrant, retarder of dissolution, accelerator of absorbtion, adhesive carrier and/or lubricant, for example, starch, mannitol, silicic acid, cellulose derivative, gelatin, alginate, glycerol, agar, calcium carbonate, sodium hydrogen carbonate, paraffin, quartarnary ammonium compound, glycerol monostearate, kaolin, bentonite, talc, potassium stearate, magnesium stearate, polyethylene glycol, and the like.

The composition may be in the form of pharmaceutically acceptable emulsion, solution, suspension, or the like.

A suppository containing the cephalosporin derivative of the invention as an active ingredient may contain polyethylene glycol and/or fatty acid or ester thereof.

A syrup or elixir may contain an inert diluent such as water and paraffin and may be used as a liquid composition suitable for oral administration. These compositions may contain an adjuvant such as wetting agent, edulcorant and seasoning agent.

The composition for injection may be a sterilized aqueous or nonaqueous solution, suspension or emulsion and may contain, for example, propylene glycol, polyethylene glycol, olive oil, and the like.

The composition may contain 0.01 to 99.5% by weight, preferably 0.1 to 90% by weight of the cephalosporin derivative of the invention as an active ingredient.

The cephalosporin derivative of the invention may be useful for the same use as the conventional cephalosporin antibiotics and effective in treating an infectious disease due to bacteria. The dose may depend on the degree of the infection and the state of the patient, and generally the dose of 0.1 to 10 g may be administered to an adult patient per one day, divided into several times.

The following Examples illustrate the present invention.

## Example 1

Preparation of N-[1,2-bis(carbomethoxy)ethyl]-7-(thienyl-2-acetamido)cephalosporanic acid amide

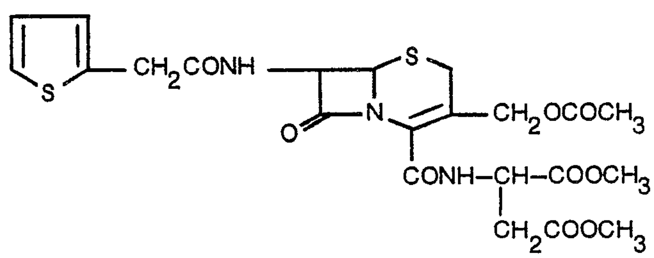

Into 100 ml of THF, 2.0 g of 7-(thienyl-2-acetamido)cephalosporanic acid, 0.8 g of dimethyl L-aspartate and 1.05 g of N,N'-dicyclohexylcarbodiimide were dissolved and stirred at room temperature for 24 hours. After the formed N,N'-dicyclohexyurea was removed by filtration, the solvent was distilled off from the filtrate and the residue was dissolved into 100 ml of chloroform. The obtained solution in chloroform was washed with 5% aqueous solution of hydrochloric acid and then with water and dried on anhydrous magnesium sulfate. After distilling off the solvent, the residue was recrystallised from a mixed solvent of ethyl acetate and n-hexane to obtain 0.41 g of crystal with the yield of 15%.

Melting point (m.p.); 160—165°C (decompose),
Infrared absorption band (I.R.) $\nu_{max}$, $cm^{-1}$ (KBr); 1782, 1730 and 1672, refer to Fig. 1,
Ultraviolet absorption band (U.V.) $\lambda_{max}$, nm ($CH_3CN$); 235 and 265,

| Elementary analysis; | C(%) | H(%) | N(%) |
|---|---|---|---|
| Calculated as $C_{22}H_{25}O_9N_3S_2$; | 48.98 | 4.64 | 7.79 |
| Found; | 49.0 | 4.6 | 7.9 |

Example 2

Preparation of N-(1-carbomethoxy-3-methylthiopropyl)-7-(thienyl-2-acetamido)cephalosporanic acid amide

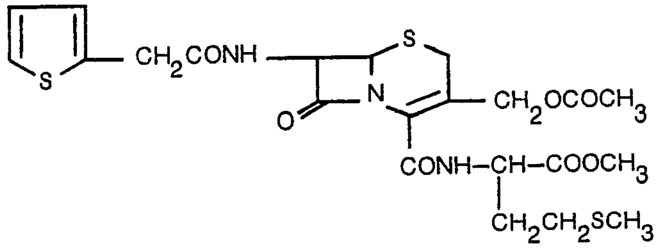

Into 100 ml of THF, 2.0 g of 7-(thienyl-2-acetamido)cephalosporanic acid, 0.82 g of methyl ester of L-methionine and 1.05 g of N,N'-dicyclohexylcarbodiimide were dissoved and stirred at room temperature for 24 hours. The produced N,N'-dicyclohexylurea was removed by filtration and the solvent was distilled off from the filtrate. The residue was dissolved into 100 ml of chloroform, washed with 5% aqueous solution of hydrochloric acid and then with water and dried on anhydrous magnesium sulfate. After distilling off the solvent, the residue was recrystallized from a mixed solvent of ethyl acetate and n-hexane to obtain 0.84 g of crystal with the yield of 31%

M.p.; 177—178°C,
I.R. $\nu_{max}$, $cm^{-1}$ (KBr); 3270, 1785, 1738, 1672, 1533 and 1225, refer to Fig. 2,
U.V. $\lambda_{max}$, nm ($CH_3CN$); 235 and 265,

| Elementary analysis; | C(%) | H(%) | N(%) |
|---|---|---|---|
| Calculated as $C_{22}H_{27}O_7N_3S_3$; | 48.80 | 4.99 | 7.76 |
| Found; | 48.7 | 5.1 | 7.8 |

Example 3

Preparation of N-[1-carboethoxy-2-(4-hydroxyphenyl)ethyl]-7-(thienyl-2-acetamido)cephalosporanic acid amide

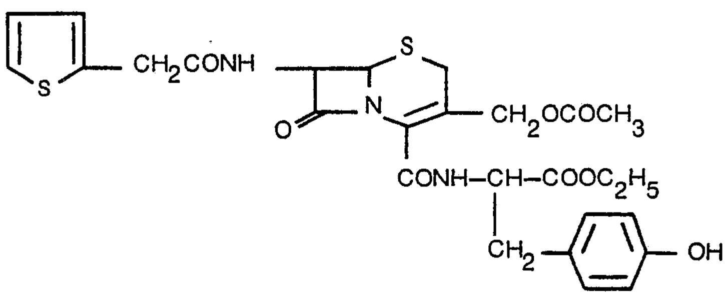

Into 100 ml of THF, 2.0 g of 7-(thienyl-2-acetamido)cephalosporanic acid, 1.05 g of ethyl ester of tyrosine and 1.05 g of N,N'-dicyclohexlylcarbodiimide were dissolved and stirred at room temperature for 24 hours. After removing the formed N,N'-dicyclohexylurea by filtration, the solvent was distilled off from the filtrate and the residue was dissolved into 100 ml of chloroform. The solution in chloroform was washed with 5% aqueous solution of hydrochloric acid and then with water and thereafter dried on anhydrous magnesium sulfate. After the solvent was distilled off, the residue was recrystallized from a mixed solvent of ethyl acetate and n-hexane to obtain 0.86 g of crystal with the yield of 29%.

M.p.; 202—203°C (decompose),
I.R. $\nu_{max}$, $cm^{-1}$ (KBr); 3300, 1798, 1738, 1671, 1540, 1524 and 1229, refer to Fig. 3,
U.V. $\lambda_{max}$, nm ($CH_3CN$); 237 and 274,

| Elementary analysis; | C(%) | H(%) | N(%) |
|---|---|---|---|
| Calculated as $C_{27}H_{25}O_7N_3S_2$; | 55.20 | 4.94 | 7.16 |
| Found; | 55.1 | 4.9 | 7.2 |

Example 4

Preparation of N-[1,3-bis(carbomethoxy)propyl]-7-(thienyl-2-acetamido)cephalosporanic acid amide

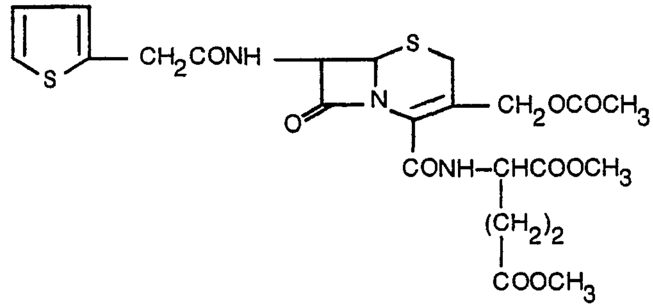

Into 10 ml of acetone was suspended 837 mg of sodium 7-(thienyl-2-acetamido)cephalosporanate. After adding 3 drops of pyridine to the suspension, 217 mg of ethyl chloroformate was added and stirred at 0°C for 30 minutes. 272 mg of dimethyl glutamate was further added to the suspension and stirred overnight at room temperature. After the reaction was over, crude crystal was obtained in the same manner as in Example 1. The obtained crude product was recrystallized from ethyl acetate/n-hexane and 535 mg of crystal was obtained with the yield of 20%.

M.p.; 124—125°C,
I.R. $\nu_{max}$, $cm^{-1}$ (KBr); refer to Fig. 4,
U.V. $\lambda_{max}$, nm ($CH_3CN$); 239 and 265,

| Elementary analysis; | C(%) | H(%) | N(%) |
|---|---|---|---|
| Calculated as $F_{23}H_{27}O_9N_3S_2$; | 51.57 | 5.08 | 7.84 |
| Found; | 51.3 | 4.8 | 7.5 |

Example 5

Preparation of N-(1-carbomethoxy-2-methylpropyl)-7-(thienyl-2-acetamido)cephalosporanic acid amide

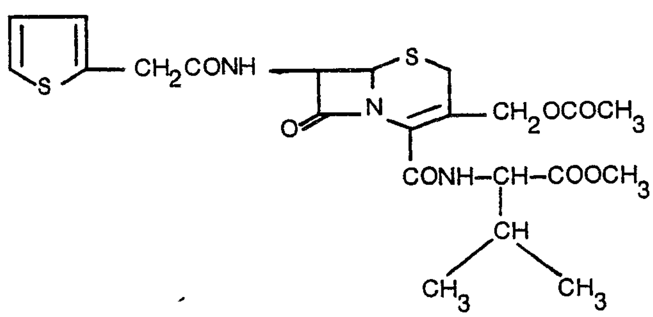

Into 10 ml of acetone was suspended 2092 mg of sodium 7-(thienyl-2-acetamido)cephalosporanate. After adding 3 drops of pyridine to the suspension, 543 mg of ethyl chlrorformate was added and stirred for 30 minutes. 838 mg of hydrochloric acid salt of valine methyl ester and 505 mg of triethylamine was further added to the suspension and stirred overnight.

After the reaction was over, the solvent was distilled off by evaporation under reduced pressure. The residue was extracted by adding 20 ml of 1% aqueous solution of $NaHCO_3$ and then ethyl acetate (3 times with 30 ml). The extract solution was washed with 30 ml of 0.01 N aqueous solution of HCl and further with 30 ml of water. The obtained solution in ethyl acetate was dried on $Na_2SO_4$, filtered on a filter paper and dried under reduced pressure. The obtained crude product was recrystallized from ethyl acetate/n-hexane and 840 mg of crystal was obtained with the yield of 33%.

M.p.; 203—204°C,
I.R. $\nu_{max}$, $cm^{-1}$ (KBr); 1793, 1755 and 1668; refer to Fig. 5,
U.V. $\lambda_{max}$, nm ($CH_3CN$); 246 and 266,

| Elementary analysis; | C(%) | H(%) | N(%) |
|---|---|---|---|
| Calculated as $C_{22}H_{27}O_7N_3S_2$; | 51.85 | 5.34 | 8.24 |
| Found; | 51.8 | 5.3 | 8.3 |

## Example 6
### Acute toxicity

Each of the cephalosporin derivarives of the invention prepared in Examples 1 to 5 was dispersed in a physiological saline solution. The dispersion was administered to ICR—JCL mice orally by a stomach sonde or intaperitoneally by injection at a predetermined amount.

After administration, the intoxication symptom was continuously observed for a week and both intravital and died mice were dissected and observed. $LD_{50}$ value was obtained from the cumulative mortality of the treated mice by applying the data to the Litchfield-Wilcoxon's graphycal method. All cephalosporin derivatives of the invention gave $LD_{50}$ value of more than 10g/kg in both oral and intraperotoneal administrations. The $LD_{50}$ value of cephalotin sodium (a conventional cephalosporin antibiotic) is about 5 g/kg.

## Example 7
### Effect on the intestinal bacterial colonies

Each of the cephalosporin derivatives of the invention prepared in Examples 1 to 5 was orally administered to mice (one group consisting of five female ICR mice of 6-week-old) for two consecutive days at a dose of 500 mg/kg/day.

Before and one day after the administration, feces of each mouse was collected and diluted with an anaerobic diluent (phosphoric buffer solution) of 100 times by volume and the diluted feces was ground. 0.1 ml of the diluted and ground feces was smeared on each culture medium shown in Table 1 and cultured aerobically or anaerobically (according to the anaerobic glove box method) under each condition shown in Table 1 and the number of each bacterium shown in Table 1 was counted.

The results are shown in Table 2.

As seen from Table 2, the number of *Escherichia coli* showed no remarkable change as compared to that before administration in the case of each of the cephalosporin derivatives of the invention, while that number increased in the case of the comparative antibiotic, cephalotin. Furthermore, *Lactobacillus acidophilus* decreased in the case of cephalotin while such a decrease was not observed in the case of the cephalosporin derivatives of the invention.

These results show that the cephalosporin derivative of the invention dose not affect the intestinal bacterial colonies.

TABLE 1
Culture medium and culture condition of bacteria

| Bacterium | Culture medium | Culture condition |
|---|---|---|
| *Escherichia coli* | DHL agar | aerobic, 37°C, one day |
| *Pseudomonas aeruginosa* | NAC agar | aerobic, 37°C, one day |
| *Streptococcus* spp | TATAC agar | aerobic, 37°C, one day |
| *Lactobacillus acidophilus* | LBS agar | anaerobic, 37°C, five days |
| *Lactobacillus bifidus* | BS agar | anaerobic, 37°C, five days |
| *Bacteroides* | NBGT agar | anaerobic, 37°C, five days |

TABLE 2

| Example No. | Logarithmic value of the number of bacterial cells per one g of feces | | | | | |
|---|---|---|---|---|---|---|
| | *E. coli* | *Ps. aeruginosa* | *Strept.* spp. | *L. acidophilus* | *L. bifidus* | *Bacteroides* |
| 1 | 6.3 | <3.3 | 7.0 | 9.0 | 8.6 | 8.5 |
| 2 | 6.4 | <3.0 | 6.6 | 8.9 | 8.3 | 8.8 |
| 3 | 6.5 | <3.0 | 6.3 | 8.9 | 8.2 | 8.7 |
| 4 | 6.0 | <3.9 | 7.0 | 9.4 | 8.9 | 8.3 |
| 5 | 6.4 | <3.0 | 6.6 | 8.9 | 8.4 | 8.6 |
| Before administration | 6.4 | <3.0 | 6.8 | 9.0 | 8.4 | 8.3 |
| Cephalotin | 9.9 | <3.0 | 9.0 | 4.1 | 9.2 | 7.2 |

Example 8

Antibacterial activity

Antibacterial activity of each of the cephalosporin derivatives of the invention prepared in Examples 1 to 5 was examined against the following two bacteria according to the standard method of Japan Society of Chemotherapy: *Escherichia coli* IFO 12734 and *Staphylococcus aureus* IAM 1011.

Each bacterial strain was inoculated into the Mueller-Hinton's culture medium and cultured at 37°C for 18 to 48 hours. The cultured medium was diluted so as to contain $1 \times 10^6$ cells of the bacteria per one ml, and the obtained medium was used as the bacterial specimen.

Agar plates were prepared by adding one part per weight of each solution of the cephalosporin derivative of the invention at a predetermined concentration to nine parts by weight of Mueller-Hinton's culture medium.

A loopful amount of the bacterial specimen prepared above was smeared to make a streak of about 2 cm on each agar plate prepared above and cultured at 37°C for 18 to 24 hours.

The minimum concentration for completely inhibiting proliferation of the bacteria (referred to as MIC) was determined.

The results are shown in Table 3.

TABLE 3

| Example No. | MIC | |
|---|---|---|
| | *E, coli* | *Staph. aureus* |
| 1 | ≥100 | ≥12.5 |
| 2 | ≥100 | ≥12.5 |
| 3 | ≥100 | ≥12.5 |
| 4 | ≥100 | 12.5 |
| 5 | ≥100 | ≥12.5 |

Example 9

The cephalosporin derivative of the invention was examined in the following experiment in order to prove that the cephalosporin derivative is activated in a living body.

As an activating enzyme of metabolism, a rat liver homogenate (S—9, manufactured by Oriental Yeast Company, Japan) was used in the following composition per 1 ml (hereinafter referred to as S—9 mix).

| | |
|---|---|
| S—9 | 0.5 ml |
| KCl | 3.3 μmol |
| $MgCl_2 \cdot 6H_2O$ | 8 μmol |
| Glucose 6-phosphate | 5 μmol |
| NADH | 4 μmol |
| NADPH | 4 μmol |
| 0.2 M phosphoric buffer solution (pH of 7.4) | 0.5 ml |

0.1 ml of the solution of each of the cephalosporin derivatives of the invention prepared in Examples 1 to 4 at a concentration of 100 μg/ml was mixed with 0.9 ml of S—9 mix or 0.9 ml of 0.1 M phosphoric buffer solution (as a control) and the obtained mixture was incubated at 37°C for 20 min with shaking.

*Staphylococcus aureus* IAM 1011 was inoculated into a Mueller-Hinton's culture medium and cultured at 37°C for 18 hours. The culture medium was adjusted to a cell concentration of $1 \times 10^8$ per 1 ml and mixed with 50 times by volume of Mueller-Hinton's agar culture medium to obtain an agar plate.

A penicillin cup of 8 mm in diameter was placed on the agar plate prepared above, and into the cup 0.1 ml of the mixture containing the cephalosporin derivative prepared above was introduced and allowed to stand at 4°C for 2 hours, and then cultured at 37°C for 18 hours to measure the diameter of a circle in which the proliferation of bacteria was inhibited (proliferation-inhibiting circle). The results are shown in Table 4. In Table 4, the proliferation-inhibiting index is shown in the ratio (%) of the diameter of the proliferation-inhibiting circle obtained by using each of the cephalosporin derivatives of the invention to that obtained by using each of the starting compound in preparing each of the cephalosporin derivatives of the invention.

| Index | % |
|---|---|
| − | 0 |
| ± | 0—1 |
| + | 1—33 |
| ++ | 33—66 |
| +++ | 66—100 |

TABLE 4

| Example No. | Proliferation-inhibiting index (%) | |
|---|---|---|
| | Before adding S—9 mix (control) | After adding S—9 mix |
| 1 | — | ± |
| 2 | — | ± |
| 3 | — | ± |
| 4 | + | ++ |

As seen from Table 4, the cephalosporin derivative of the invention is activated by the action of an enzyme in a living body to recover the antibacterial activity, although the cephalosporin derivative of the invention per se shows a low antibacterial activity in the absence of an activating enzyme.

Example 10

*Escherichia coli* IFO 12734 (1.4 × $10^8$ cells) was inoculated intraperitoneally to ddY—SPF mice (a group consisting of 20 mice). Just after and 4 hours after the infection, each of the cephalosporin derivatives of the invention prepared in Examples 1 to 5 was administered orally at a dose of 500 mg/kg, and the mortality of the mice due to infection was observed for 7 days. More than 35% of the mice administered with the cephalosporin derivative of the invention survived even on the 7th day after the infection, although all mice without administration of the cephalosporin derivative of the invention died on 2nd day after infection.

The results show that the cephalosporin derivative of the invention is effective for oral administration against an infectious disease.

Example 11

*Formulation*

(1) Tablet

A tablet was prepared by a following composition in one tablet of 200 mg;

| | |
|---|---|
| the cephalosporin derivative prepared in Example 1 | 175 mg |
| lactose | 16 mg |
| starch | 5 mg |
| hydroxypropylcellulose | 3 mg |
| magnesium stearate | 1 mg |

The cephalosporin derivative of the invention and lactose was mixed and then an aqueous solution of hydroxypropylcellulse was admixed, and the mixture was kneaded, dried and pulverized. Then magnesium stearate preliminarily dispersed into starch was admixed and the mixture was tabletted by the conventional method.

(2) Granule

A granule was prepared from a following composition;

| | |
|---|---|
| the cephalosporin derivative prepared in Example 2 | 176 mg |
| lactose | 16 mg |
| starch | 4 mg |
| hydroxypropylcellulose | 4 mg |

The cephalosporin derivative of the invention, starch and lactose were mixed, and an aqueous solution of hydroxycellulose was then admixed, and the mixture dried and pulverized. The pulverized material was sifted by 12 to 48 mesh sieves to obtain a granule.

## Claims

1. A cephalosporin derivative having the general formula (I):

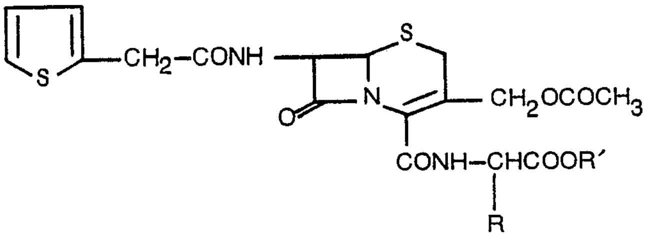

(I)

wherin R' is a hydrogen atom, an alkyl group having 1 to 4 carbon atoms or a pharmaceutically acceptable cation and R is

$-CH_2COOR'$, $-CH_2CH_2SR'$, 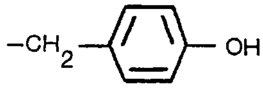, $-CH_2CH_2COOR'$, or $-CH(CH_3)_2$

wherein R' is as defined above.

2. A derivative according to claim 1 wherein R' is an alkali metal cation.

3. A derivative according to claim 1, which is N-[1,2-bis(carbomethoxy)ethyl]-7-(thienyl-2-acetamido)cephalosporanic acid amide.

4. A derivative according to claim 1, which is N-(1-carbomethoxy-3-methylthiopropyl)-7-(thienyl-2-acetamido)cephalosporanic acid amide.

5. A derivative according to claim 1, which is N-[1-carboethoxy-2-(4-hydroxyphenyl)ethyl]-7-(thienyl-2-acetamide)cephalosporanic acid amide.

6. A derivative according to claim 1, which is N-[1,3-bis(carbomethoxy)propyl]-7-(thienyl-2-acetamido)cephalosporanic acid amide.

7. A derivative according to claim 1, which is N-(1-carbomethoxy-2-methylpropyl)-7-(thienyl-2-acetamido)cephalosporanic acid amide.

8. A process for preparing a cephalosporanic derivative as claimed in claim 1, which process comprises reacting 7-(thienyl-2-acetamido)cephalosporanic acid having the formula (II):

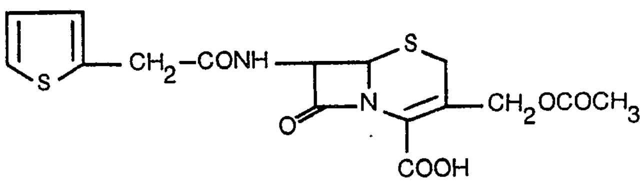

(II)

or a salt or a chloride thereof, with a compound having the general formula (III):

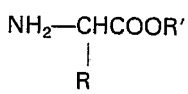

(III)

wherein R and R' are as defined in claim 1.

9. A process according to claim 8, wherein the reaction is carried out in an organic solvent at the temperature of from −30°C to 50°C for from 0.5 to 48 hours.

10. A process according to claim 8 or 9, wherein the reaction is carried out under the presence of an activating agent selected from carbodiimide, ethyl chloroformate and oxyalyl chloride.

11. A pharmaceutical composition comprising, as active ingredient, a cephalosporin derivative as claimed in any one of claims 1 to 7 or which has been prepared by a process as claimed in any one of claims 8 to 10, together with a pharmaceutically acceptable carrier, diluent or adjuvant.

12. A pharmaceutical composition according to claim 11 in unit dosage form.

**Patentansprüche**

1. Cephalosporinderivat mit der allgemeinen Formel (I):

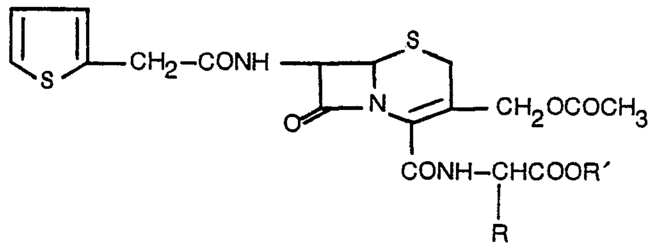

(I)

worin R′ ein Wasserstoffatom, eine Alkylgruppe mit 1 bis 4 Kohlenstoffatomen oder ein pharmazeutisch verträgliches Kation ist und R

$-CH_2COOR'$, $-CH_2CH_2SR'$, $-CH_2$ 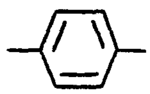 OH, $-CH_2CH_2COOR'$ oder $-CH(CH_3)_2$ ist,

worin R′ wie oben definiert ist.

2. Derivat nach Anspruch 1, worin R′ ein Alkalimetallkation ist.

3. Derivat nach Anspruch 1, welches N-[1,2-bis(Carbomethoxy)ethyl]-7-(thienyl-2-acetamido)-cephalosporansäueramid ist.

4. Derivat nach Anspruch 1, welches N-(1-Carbomethoxy-3-methylthiopropyl)-7-(thienyl-2-acetamido)cephalosporansäureamid ist.

5. Derivat nach Anspruch 1, welches N-[1-Carboethoxy-2-(4-hydroxyphenyl)ethyl]-7-(thienyl-2-acetamid)cephalosporansäureamid ist.

6. Derivat nach Anspruch 1, welches N-[1,3-bis(Carbomethoxy)propyl]-7-(thienyl-2-acetamido)-cephalosporansäureamid ist.

7. Derivat nach Anspruch 1, welches N-(1-Carbomethoxy-2-methylpropyl)-7-(thienyl-2-acetamido)-cephalosporansäureamid ist.

8. Verfahren zur Herstellung eines Cephalosporanderivats nach Anspruch 1, wobei das Verfahren die Reaktion von 7-(Thienyl-2-acetamido)cephalosporansäure mit der Formel (II):

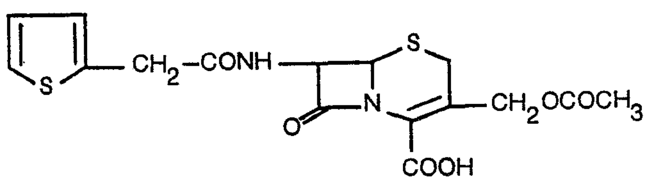

(II)

oder einem Salz oder einem Chlorid davon mit einer Verbindung mit der allgemeinen Formel (III):

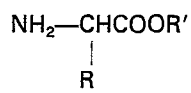

(III)

worin R und R′ wie in Anspruch 1 definiert sind, umfaßt.

9. Verfahren nach Anspruch 8, worin die Reaktion in einem organischen Lösungsmittel bei einer Temperatur von −30°C bis 50°C während 0,5 bis 48 Studen durchgeführt wird.

10. Verfahren nach Anspruch 8 oder 9, worin die Reaktion unter Gegenwart eines Aktivierungsmittels, ausgewählt aus Carbodiimid, Ethylchloroformat und Oxyalylchlorid, durchgeführt wird.

11. Pharmazeutische Zusammensetzung, die als Wirkstoff ein Cephalosporinderivat nach einem der Ansprüche 1 bis 7 umfaßt, oder die durch ein Verfahren nach einem der Ansprüche 8 bis 10 hergestellt wird, zusammen mit einem pharmazeutisch verträglichen Trägerstoff, Verdünnungsmittel oder Adjuvans.

12. Pharmazeutische Zusammensetzung nach Anspruch 11 in Form einer Dosierungseinheit.

**Revendications**

1. Un dérivé de céphalosporin répondant à la formule générale (I):

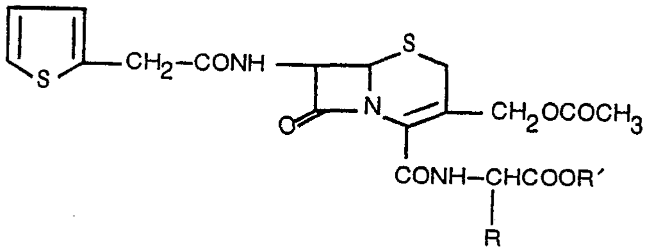

(I)

dans laquelle R′ est un atome d'hydrogène, un groupe alcoyle ayant 1 à 4 atomes de carbone ou un cation convenant en pharmacie et R est

$-CH_2COOR'$, $-CH_2CH_2SR'$, 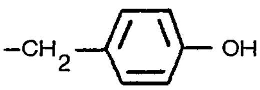 , $-CH_2CH_2COOR'$, or $-CH(CH_3)_2$

où R′ est comme défini ci-dessus.

2. Un dérivé selon la revendication 1 où R′ est un cation de métal alcalin.

3. Un dérivé selon la revendication 1 qui est l'amide de l'acide N-[1,2-bis(carbométhoxy)éthyl]-7-(thiényl-2-acétamido)céphalosporanique.

4. Un dérivé selon la revendication 1 qui est l'amide de l'acide N-(1-carbométhoxy-3-méthylthiopropyl)-7-(thiényl-2-acétamido)céphalosporanique.

5. Un dérivé selon la revendication 1 qui est l'amide de l'acide N-[1-carboéthoxy-2-(4-hydroxyphényl)éthyl]-7-(thiényl-2-acétamido)céphalosporanique.

6. Un dérivé selon la revendication 1 qui est l'amide de l'acide N-[1,3-bis(carbométhoxy)propyl]-7-(thiényl-2-acétamido)céphalosporanique.

7. Un dérivé selon la revendication 1 qui est l'amide de l'acide N-(1-carbométhoxy-2-méthylpropyl)-7-(thiényl-2-acétamido)céphalosporanique.

8. Un procédé pour la préparation d'un dérivé céphalosporanique comme revendiqué dans la revendication 1, lequel procédé comprend la réaction de l'acide 7-(thiényl-2-acétamido)céphalosporanique de formule (II):

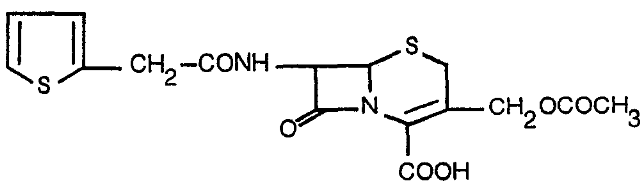

(II)

ou d'un sel ou d'un chlorure de celui-ci, avec un composé répondant à la formule générale (III):

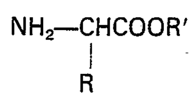

(III)

dans laquelle R et R′ sont comme défini dans la revendication 1.

9. Un procédé selon la revendication 8 dans lequel la réaction est effectuée dans un solvant organique à une température de −30°C à 50°C pendant 0,5 à 48 heures.

10. Un procédé selon la revendication 8 ou 9, dans lequel la réaction est effectuée en présence d'un agent d'activation choisi parmi un carbodiimide, le chloroformiate d'éthyle et le chlorure d'oxalyle.

11. Une composition pharmaceutique comprenant comme ingrédient actif un dérivé de céphalosporine comme revendiqué dans l'une quelconque des revendications 1 à 7 ou qui a été préparé selon un procédé comme revendiqué dans l'une quelconque des revendications 8 à 10, avec un support, diluant ou adjuvant convenant en pharmacie.

12. Une composition pharmaceutique selon la revendication 11 sous forme d'une dose unitaire d'administration.

FIG.1

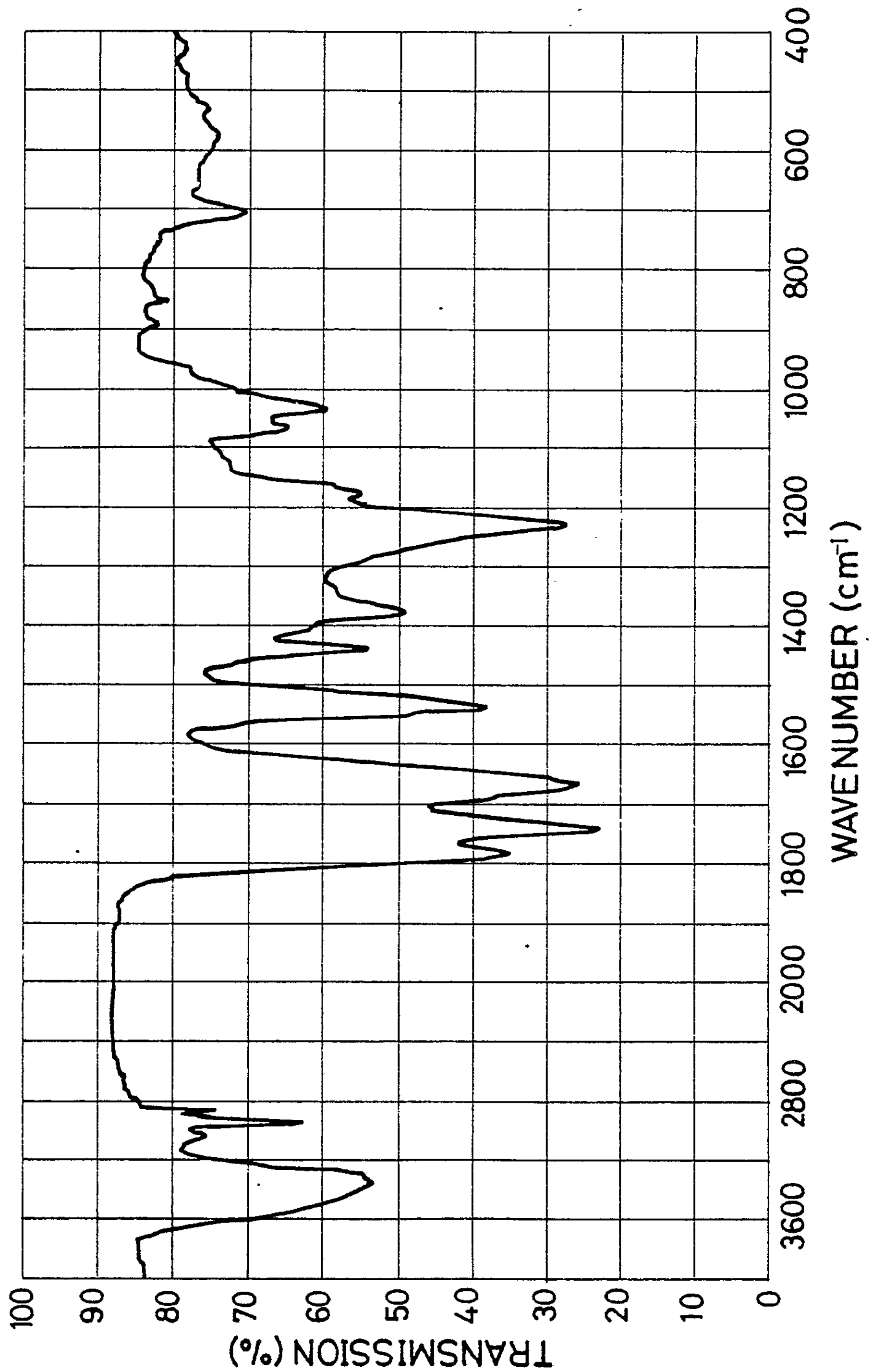
TRANSMISSION (%)
100
90
80
70
60
50
40
30
20
10
0
3600
2800
2000
1800
1600
1400
1200
1000
800
600
400
WAVENUMBER (cm⁻¹)

FIG. 2

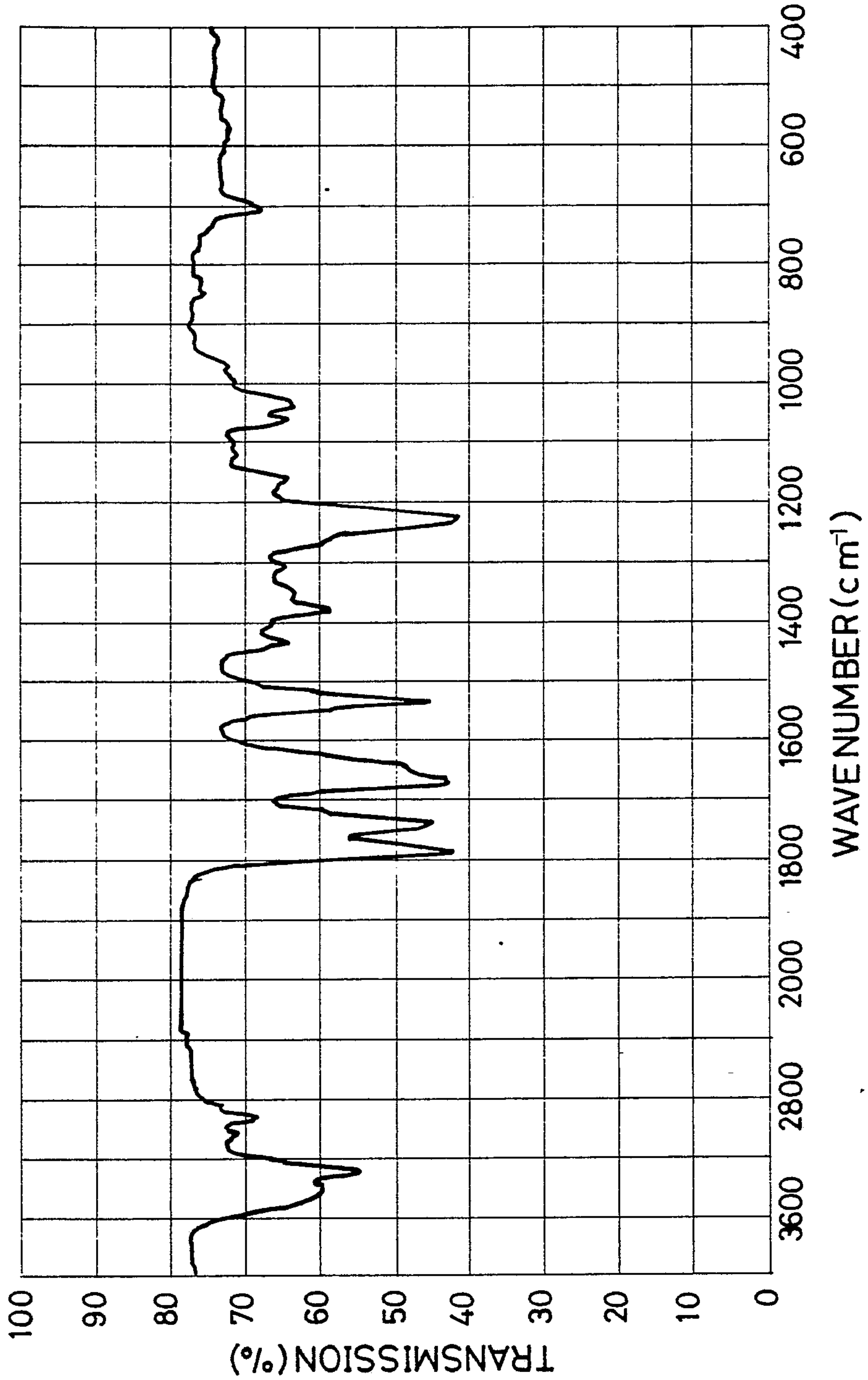
TRANSMISSION (%)
100
90
80
70
60
50
40
30
20
10
0
WAVE NUMBER (cm$^{-1}$)
3600
2800
2400
2000
1800
1600
1400
1200
1000
800
600
400

FIG.3

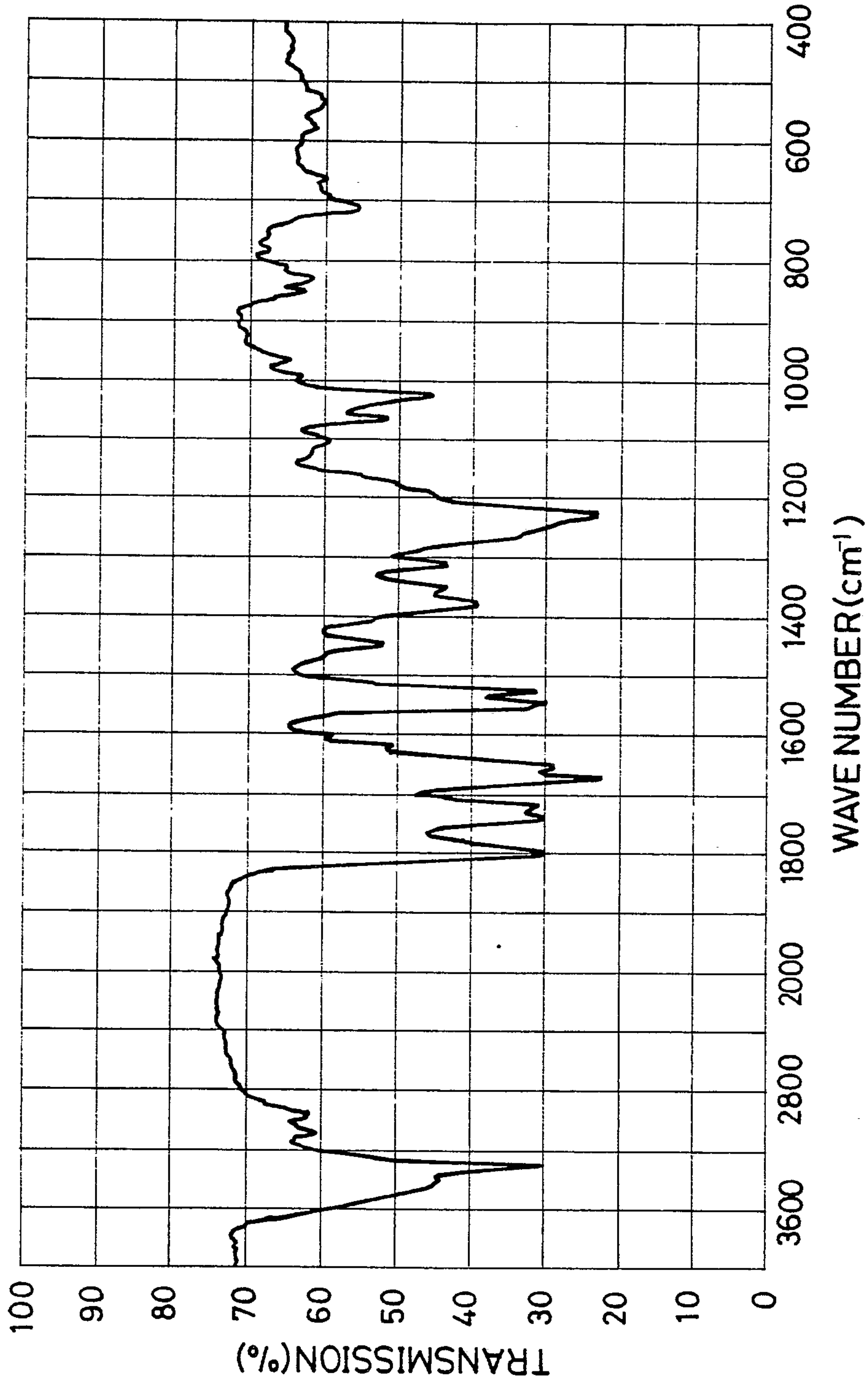

TRANSMISSION (%)
100
90
80
70
60
50
40
30
20
10
0
3600
2800
2000
1800
1600
1400
1200
1000
800
600
400
WAVE NUMBER (cm⁻¹)

FIG.4

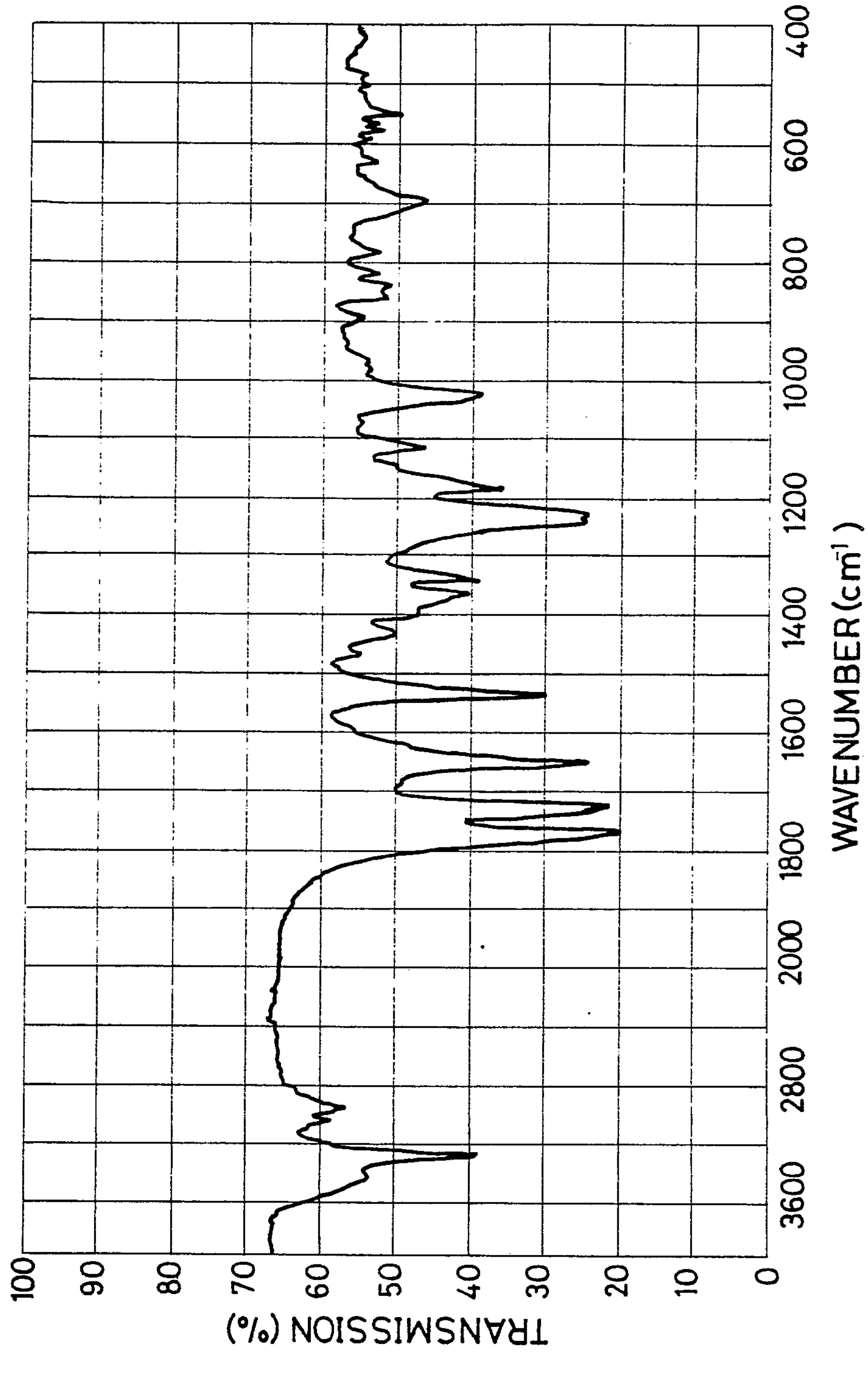
TRANSMISSION (%)
100
90
80
70
60
50
40
30
20
10
0
3600
2800
2000
1800
1600
1400
1200
1000
800
600
400
WAVENUMBER (cm⁻¹)

FIG. 5

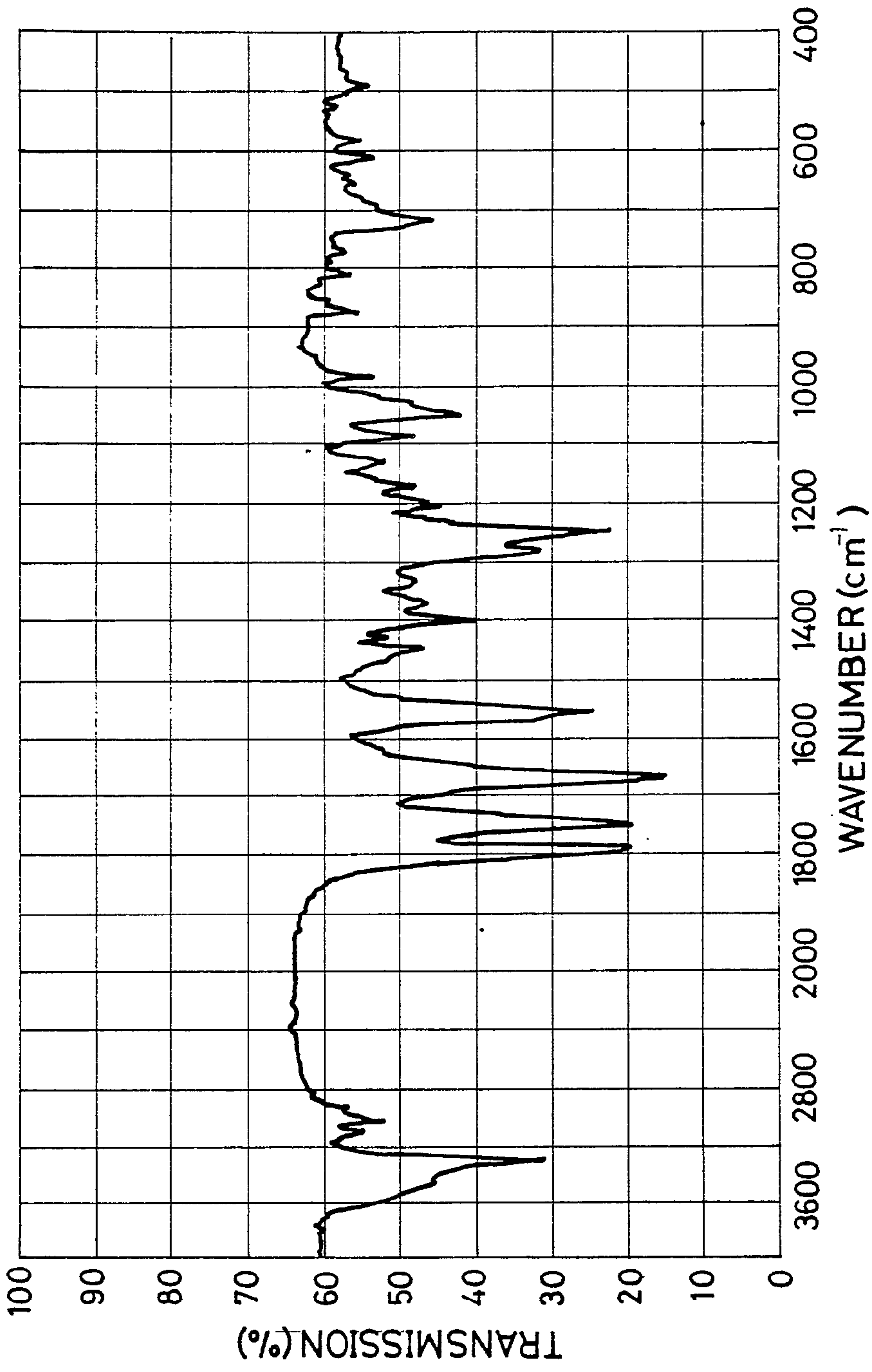
TRANSMISSION (%)
100
90
80
70
60
50
40
30
20
10
0
WAVENUMBER (cm$^{-1}$)
3600
2800
2000
1800
1600
1400
1200
1000
800
600
400